# EUROPEAN PATENT SPECIFICATION

(11) **EP 1 210 409 B1**
(45) Date of publication and mention of the grant of the patent: **29.11.2006**
(21) Application number: 00958809.6
(22) Date of filing: 07.09.2000
(51) Int. Cl.: C12N 5/00, A61L 27/38, C03B 37/00, C03C 3/16

(54) **CELL GROWTH SUBSTRATE**
TRÄGERMATERIAL ZUM WACHSEN VON ZELLEN
SUBSTRAT DE CROISSANCE POUR DES CELLULES

(30) Priority: 07.09.1999 GB 9920974; 25.11.1999 GB 9927802
(43) Date of publication of application: 05.06.2002
(73) Proprietor: Tyco Healthcare Group LP, Mansfield, MA 02048 (US)
(72) Inventor: GILCHRIST, Thomas, Ayr KA7 2TW (GB); HEALY, David Michael, Ayr KA7 4EG (GB)
(74) Representative: Ouzman, Beverley Nicola Claire
(86) International application number: PCT/GB2000/003424
(87) International publication number: WO 2001/018174

(56) References cited:
- WO-A-98/54104
- US-A- 5 811 302
- BURNIE J ET AL: "CONTROLLED RELEASE GLASSES (C.R.G.) FOR BIOMEDICAL USES" BIOMATERIALS,GB,ELSEVIER SCIENCE PUBLISHERS BV., BARKING, vol. 2, 1 October 1981 (1981-10-01), pages 244-246, XP000650239 ISSN: 0142-9612
- SALIH, V. ET AL: "The response of human osteoblast cell lines to phosphate-based soluble glasses" BIOCERAM., PROC. INT. SYMP. CERAM. MED. (1998), 11, 269-272 , 1998, XP000990696
- UO MOTOHIRO ET AL: "Properties and cytotoxicity of water soluble Na2O-CaO-P2O5 glasses." BIOMATERIALS, vol. 19, no. 24, December 1998 (1998-12), pages 2277-2284, XP004168861 ISSN: 0142-9612

## Description

The present invention provides a growth substrate for cell culture. In particular, the present invention provides a cell culture growth substrate for tissue engineering.

Tissue engineering is expected to transform orthopaedics treatments, cancer therapy and the treatment of chronic degenerative diseases. Tissue engineering concerns the provision of a graft comprising living cells or suitable substrate to sustain the growth of such cells which integrate into the patient providing expedited wound healing and repair or an alternative drug delivery or gene therapy delivery system. The tissue engineering graft may be an autograft, allograft or xenograft. Autografts are formed with the patient's own cells, cultured with a suitable growth medium or substrate. Allografts rely upon cells donated from an alternative same species source (including cadaver or foetal sources) whilst xenografts rely upon cells donated from other species.

Both allografts and xenografts may be treated to minimise autoimmune rejection of the graft following implantation.

There are numerous potential applications for tissue engineered grafts, including reconstructive surgery, orthopaedics or dental applications, burn treatments or ulcer treatments (including venous ulcers and diabetic foot ulcers). A number of tissue engineered grafts have been described in the literature (see Dutton, "Tissue Engineering", Genetic Engineering News, Vol 18, No 8, April 15, 1998).

Implants containing controlled release glass were disclosed in "Controlled Release Glasses (C.R.G.) for biomedical uses" authored by J. Burnie et al. (Biomaterials, GB, Elsevier Science Publishers BV., Barking, Vol. 2, 1 October 1981 - page 244-46 ISSN: 0142-9612). J. Burnie et al. do not disclose a single specific controlled release glass composition, however, and do not provide an enabling disclosure.

Examples of such tissue engineered grafts include APLIGRAF (Trade Mark) which is a bilayer graft including both differentiated keratinocytes and a layer of fibroblasts in a collagen matrix. APLIGRAF has been used as a skin graft, particularly for burns, diabetic foot ulcers, excisional surgery and venous ulcers (Bender, "Healing of Difficult to Heal Wounds Using a Bilayered Skin Construct", 11^{th} Annual Symposium on Critical Issues in Surgery-Wound Healing, Science and Technology, 3-5 December 1998, St Thomas, US Virgin Islands). Other bioengineering skin equivalents include INTEGRA (Trade Mark), a xenograft of bovine collagen, glycosamionglycan (GAG) and silastic sheet; ALLODERM (Trade Mark), an allograft of treated cadaver skin; and DERMOGRAFT (Trade Mark), an allograft of neonatal fibroblasts on a polyglactin scaffold. Tissue engineered grafts for bone include RAINBOW (Trade Mark) of IsoTis VB which is a biomimetic coating which allows a bone-like layer to grow over metal prosthesis and serves as a scaffold for bone growth, and also EMBARC (Trade Mark) which is a resorbable bone repair material.

Despite the numerous tissue engineering grafts currently being developed, there is still a demand for further and improved products. We have now found that water-soluble glass acts as a support or matrix for cell growth and hence the glass has utility in tissue engineering.

The present invention thus provides a cell culture growth substrate comprising a water-soluble glass matrix adapted to sustain the growth of living cells. The substrate will comprise or have at least a portion of the surface thereof coated with living cells, wherein the glass of said water-soluble glass matrix comprises at least one metallic ion or boron containing compound.

In one embodiment the cell culture growth substrate is pre-seeded with living cells and hence the matrix comprises or has at least a portion of its surface coated with living cells.

In one embodiment, the cell culture growth substrate will be useful as an implant or tissue graft, i.e. is designed for implantation into a patient to replace or promote repair of damaged tissues.

The water-soluble glass matrix will of course be biocompatible. Generally, the biodegradation of the water-soluble glass following implantation of the graft into a patient will be pre-determined to be compatible with the timescale required for regrowth of the tissues concerned.

The glass present in the graft acts as a cell support matrix and will function as such in vivo. Thus the graft can be used directly in vivo to provide a temporary biodegradable scaffold which will encourage ingrowth of surrounding tissues. In other embodiments pre-seeding of the graft with a pre-selected cell type, and optionally growth of that cell type, prior to implantation may be desirable.

In an alternative embodiment, the cell culture growth substrate is intended for non-clinical purposes, for example in bio-reactor and fermentation technologies for the production of drugs and other biologically derived chemicals. Organisms usually grow with increased confluence on surfaces, and enzyme reactions (and many other biochemical reactions) are generally most efficient when the enzyme is bound to a reaction surface. Beads, sinters and fibres can be used to provide the required mechanical support, with large (productive) surface areas and additional features such as controlled inorganic micro-nutrient supply, contamination control, pH buffering and a biocompatible carrier which will allow the subsequent transfer or filtration of cells, enzymes or other components bound to its surface on completion of the reaction stage.

Conveniently the water-soluble glass matrix may be in the form of water-soluble glass fibres and reference is made to our WO-A-98/54104 which describes the production of suitable glass fibres. Whilst the glass fibres can be used in the form of individual strands, woven (e.g. a 1 x 1 basket weave) or non-woven mats may also be produced from the fibres and used as the matrix. The individual fibres of a non-woven mat may be gently sintered together to obtain coherence of the strands. Alternatively, the fibres may be used as glass wool and this form of matrix is especially suitable where the graft requires a 3D shape.

Alternatively, the water-soluble glass matrix may be produced from finely comminuted glass particles. For example, the particles may have an average diameter of from 15 µm to 6 mm, preferably from 50µm to 6 mm. Optionally, the glass particles may be sintered together to form a porous structure into or onto which cells may be seeded and in this embodiment the glass particles will have a preferred diameter of from 53 µm to 2 mm, preferably 400 µm to 2 mm. Again, a three-dimensionally shaped graft may be produced (if necessary individually tailored to be compatible with the wound site of the patient) from the sinter. Alternatively, particles following a Fuller curve packing distribution and having a range of diameters of 0.3 mm to 5.6 mm may be used.

In a further embodiment the glass may simply be in the form of a glass sheet, which may be substantially planar or may be contoured to a required shape. Etched, ground or patterned glass sheet may be used in addition to plain surfaced glass.
The water-soluble glass preferably includes phosphorous pentoxide (P₂O₅) as the glass former.

The water-soluble glass matrix comprises at least one metallic ion or boron containing compound.

Generally the mole percentage of phosphorous pentoxide in the glass composition is less than 85%, preferably less than 60% and especially between 30-60%.

One or more oxides or carbonates of alkali, alkaline earth and transition metals are preferably used as glass modifiers.

Generally, the mole percentage of these oxides or carbonates of alkali, alkaline earth and transition metals is less than 60%, preferably between 40-60%.

Boron containing compounds (e.g. B₂O₃) are preferably used as glass additives.

Generally, the mole percentage of boron containing compounds is less than 15% or less, preferably less than 5%.

Other compounds may also be added to the glass to modify its properties, for example SiO₂, Al₂O₃, SO₃, sulphate ions (SO₄²⁻) or transition metal compounds (e.g. first row transition metal compounds).

Typically the soluble glasses used in this invention comprise phosphorus pentoxide (P₂O₅) as the principal glass-former, together with any one or more glass-modifying non-toxic materials such as sodium oxide (Na₂O), potassium oxide (K₂O), magnesium oxide (MgO), zinc oxide (ZnO) and calcium oxide (CaO). The rate at which the glass dissolves in fluids is determined by the glass composition, generally by the ratio of glass-modifier to glass-former and by the relative proportions of the glass-modifiers in the glass. By suitable adjustment of the glass composition, the dissolution rates in water at 38°C ranging from substantially zero (i.e. just above zero) to 25mg/cm²/hour or more can be designed. However, the most desirable dissolution rate R of the glass is between 0.001 and 2.0mg/cm²/hour.

The water-soluble glass is preferably a phosphate glass, and comprises a source of metal ions or boron which confer either antimicrobial protection or enhanced cell growth, or both, or which are useful trace elements. Examples include silver, copper, magnesium, zinc, iron, cobalt, molybdenum, chromium, manganese, cerium, selenium, and these metal ions can be included singly or in any combination with each other. Where silver ions are of interest, these may advantageously be introduced during manufacture as silver orthophosphate (Ag₃PO₄). The glass preferably enables controlled release of metal ions or boron and other constituents in the glass and the content of these additives can vary in accordance with conditions of use and desired rates of release, the content of silver generally being up to 5 mole %. While we are following convention in describing the composition of the glass in terms of the mole % of oxides, of halides and of sulphate ions, this is not intended to imply that such chemical species are present in the glass nor that they are used for the batch for the preparation of the glass.

The optimum rate of release of metal ions into an aqueous environment may be selected by circumstances and particularly by the specific function of the released metal ions. The invention provides a means of delivering metal ions or boron to an aqueous medium at a rate which will maintain a concentration of metal ions or boron in said aqueous medium of not less than 0.01 parts per million and not greater than 10 parts per million. In some cases, the required rate of release may be such that all of the metal added to the system is released in a short period of hours or days and in other applications it may be that the total metal be released slowly at a substantially uniform rate over a period extending to months or even years. In particular cases there may be additional requirements, for example it may be desirable that no residue remains after the source of the metal ions is exhausted or, in other cases, where the metal is made available it will be desirable that any materials, other than the metal ions itself, which are simultaneously released should be physiologically harmless. In yet other cases, it may be necessary to ensure that the pH of the resulting solution does not fall outside defined limits.

Generally, the mole percentage of these additives in the glass is less than 25%, preferably less than 10%.

The cells may be any suitable cells required for grafts. Particular mention may be made of keratinocytes, fibroblasts, chrondrocytes and the like as preferred cell types. Mention may also be made of stem cells (mesenchymal, haematopoetic, and embryonic), Schwaan cells, keratinocytes (epithelial cells), chondrocytes, osteoblasts, endothelial cells and other fibroblasts, cardiac cells (and other myoblasts), pancreatic β cells and periodontal tissues e.g. Dentine, but the invention is not limited to these cell types alone.

Embodiments of the invention will be described with reference to the following non-limiting examples and Figs. in which:
Fig. 1
   Chondrocytes forming a monolayer on a glass fibre (Example 1) as viewed by laser scanning confocal microscope.
Fig. 2
   Fluorescent microscopy of HUE cells on MATT01 glass fibres (see Example 2).
Fig. 3
   Fluorescent microscopy of HUE cells on MATT04 glass fibres (see Example 2) .
Fig. 4
   SEM picture of L929 cells on glass surface at x30 magnification (see Example 3).
Fig. 5
   SEM picture of L929 cells on glass surface at x170 magnification (see Example 3).
Fig. 6
   SEM picture of L929 cells on glass surface at x215 magnification (see Example 3).
Fig. 7
   SEM picture of L929 cells on glass surface at x610 magnification (see Example 3).
Fig. 8
   Bar chart showing cell activity vs. concentration for Ag/Mg in a PBS Extraction Vehicle (see Example 4).
Fig. 9
   Bar chart showing cell activity vs. concentration for Ag/Ni in a PBS Extraction Vehicle (see Example 4).
Fig. 10
   Bar chart showing cell activity vs. concentration for Ag/Zn in an MEM Extraction Vehicle (see Example 4).
Fig. 11
   Bar chart showing cell activity vs. concentration for Ag/B in a PBS Extraction Vehicle (see Example 4).
Fig. 12a
   Bar chart showing cell activity vs. concentration for Mg/Cu in a PBS Extraction Vehicle (see Example 4).
Fig. 12b
   Bar chart showing cell activity vs. concentration for Mg/Cu in an MEM Extraction Vehicle (see Example 4).
Fig. 13
   Bar chart showing cell activity vs. concentration for Mg/Ni in a PBS Extraction Vehicle (see Example 4).
Fig. 14a
   Bar chart showing cell activity vs. concentration for Mg/B in a PBS Extraction Vehicle (see Example 4).
Fig. 14b
   Bar chart showing cell activity vs. concentration for Mg/B in a MEM Extraction Vehicle (see Example 4).
Fig. 15a
   Bar chart showing cell activity vs. concentration for Ni/Cu in a PBS Extraction Vehicle (see Example 4).
Fig. 15b
   Bar chart showing cell activity vs. concentration for Ni/Cu in a MEM Extraction Vehicle (see Example 4).
Fig. 16
   Bar chart showing cell activity vs. concentration for Ni/Zn in a PBS Extraction Vehicle (see Example 4).
Fig. 17a
   Bar chart showing cell activity vs. concentration for Ni/B in a PBS Extraction Vehicle (see Example 4).
Fig. 17b
   Bar chart showing cell activity vs. concentration for Ni/B in a MEM Extraction Vehicle.
Fig. 18
   Bar chart showing cell activity vs. concentration for Cu/Zn in a MEM Extraction Vehicle.
Fig. 19
   Bar chart showing cell activity vs. concentration for Cu/B in a MEM Extraction Vehicle.
Fig. 20
   Bar chart showing cell activity vs. concentration for Zn/B in a PBS Extraction Vehicle.

### Example 1

### Introduction

Controlled Release Glass (CRG) is a phosphate-based material which degrades at a predeterminable rate. The potential for using CRG as a cartilage engineering matrix has been assessed using isolated equine chondrocytes with *in-vitro* techniques. The glass was provided in fibrous form in three different compositions. The three CRG compositions provided have shown potential as a tissue engineering substrate.

### Materials and Method

A total of 200,000 chondrocytes isolated from horse articular cartilage were added to each 2 cm well in a 24 well plate. Every well contained 0.02 grams of glass fibre sample. Four different fibres F1 to F4 (diameters 20-30 µm) were analysed: F1 - containing Fe₂O₃ and NaF, F2 - containing Ce₂O₃ and Se. The composition of glasses used to form F1 to F4 are set out below in Table 1. The culture medium (containing 10% FCS) was changed daily. At time periods of 3 days, 1 week and 2 weeks, the samples were stained using rhodamine phalloidin and oregan green for the viewing of actin and tubulin using a laser scanning confocal microscope. At the same time periods, the cell supernatant was removed and stored at -80°C until analysis on cell viability and type II collagen production could be performed. Production of type II collagen was analysed by using RT-PCR analysis on the cDNA from the chondrocyte population in contact with the glass fibres. The total RNA was prepared from the cell population by the addition of 1 ml of TRIzol (SIGMA) to the cell population for 5 minutes. After this time, the TRIzol was retrieved and stored at -80°C until RT-PCR analysis could be carried out. The RT-PCR analysis was performed by tagging with primers for collagen type II and with gapDH for cell viability.

Zymography was also performed at time periods of 4 days, 1 week and 2 weeks for detection of matrix metalloproteinases (MMP's) produced by the chondrocytes.

**Table 1**

| **BATCH RECORD SELECTION** | | | | | | | | | | | | | | | | |
|---|---|---|---|---|---|---|---|---|---|---|---|---|---|---|---|---|
| Code | **Formulation as mole%** | | | | | | | | | | | | | **Solution Rates** | | **Physical Form** |
| | **Na**₂**O** | **CaO** | **Ag**₂**O** | **P**₂**O**₅ | **MgO** | **K**_{**2**}**O** | **B**_{**2**}**O**_{**3**} | **MnO** | **Fe**_{**2**}**O**_{**3**} | **NaF** | **Ce**_{**2**}**O**_{**3**} | **Se** | **TOTAL** | **Annealed @ 37.5°C (mg. cm**^{**-2**}**. hr**^{**-1**}**)** | **Non-Annealed @ 37.5°C (mg.cm**^{**-2**}**.hr**^{**-1**}**)** | |
| F1 | 25.85 | 17.26 | | 46.78 | | | 5.75 | 1.5 | 1.76 | 1.1 | | | 100 | N/A | N/A | F,C + R |
| F2 | 25.78 | 17.42 | | 46.1 | | | 5.82 | 1.52 | 0.95 | | 2.01 | | 99.6 | N/A | N/A | F,C + R |
| F3 | 25.19 | 17.03 | | 45.05 | | | 5.68 | 1.49 | 0.93 | 0.4 | 1.96 | 2.27 | 100 | N/A | N/A | F,R + C |
| F4 | 32 | | 3 | 46 | 4 | 10 | 5 | | | | | | 100 | 0.331 | 0.9614 | F + R |

| | | | | | | | | | | | | | | | | |
|---|---|---|---|---|---|---|---|---|---|---|---|---|---|---|---|---|
| F=fibres; C=cullet; R=rods | | | | | | | | | | | | | | | | |

### Results and Conclusions

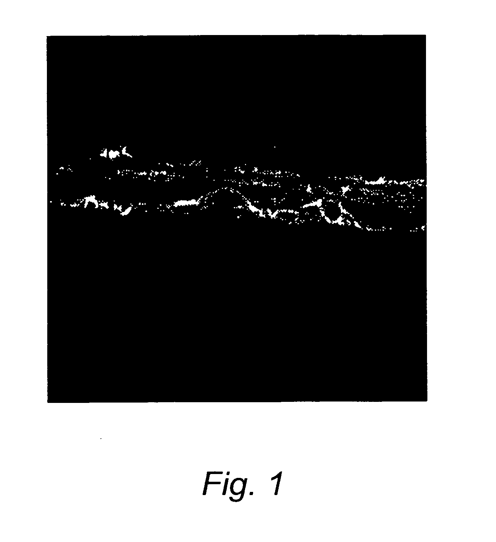

Chondrocytes adhered to all three types of fibre sample. At the 3 day time period, the cells appeared to be rounded. At 1 week and 2 weeks, confocal microscopy indicated cell proliferation between all time periods. At 1 week and 2 weeks, the cells were elongated and formed a monolayer along the fibre length as can be seen in Fig. 1.

The RT-PCR analysis showed that fibres F2 and F3 were producing collagen type II up to and including the two week time period indicating that the cells retained their chondrocytic phenotype.

The zymography performed on F2 and F3 showed that the cells in contact with these fibres produced MMP2 at all three time periods, but in a greater quantity at 2 weeks than 1 week, and at 1 week than 4 days. This increase of MMP2 production is expected, as the cells were seen to have increased in number at these time periods from the confocal microscope analysis.

In conclusion, all three fibres types showed cell adherence and the chondrocytes adhered to F2 and F3 appear to retain the ability to produce type II collagen.

### Example 2

### Biological Evaluation of Non-woven Mat Fibres

### 1. Objective

Using in-vitro techniques determine:
a. The cytotoxicity of a series of five non-woven mat CRG fibres.
b. The potential of the fibres as a cell substrate matrix.

### 2. Scope

The test procedures apply to all fibre samples.

### 3. Equipment and Materials

3.1 Equipment
3.1.1 Laminar air flow hood
3.1.2 Incubator maintained at 37°C/5% carbon dioxide
3.1.3 Refrigerator at 4°C
3.1.4 Freezer at -18°C
3.1.5 Vacuum source
3.1.6 Phase contrast microscope

3.2 Materials
3.2.1 Sterile plastic-ware pipettes
3.2.2 Sterile glass pipettes
3.2.3 24 well Sterile dishes
3.2.4 Surgical grade forceps
3.2.5 Surgical grade scissors
3.2.6 Sterile Universal containers
3.2.7 L929 cell culture line (ATCC NCTC Clone 929)
3.2.8 Human Umbilical endothelial cells (primary cell source, Liverpool Women's Hospital)
3.2.9 TCPS negative control
3.2.10 CRG fibres:
   D021298F1 (MATT01)
   D301198F1 (MATT02)
   D100299F1 (MATT03)
   D161298F2 (MATT04)
   D171298F2 (MATT05)
   All CRG fibres were supplied non-sterile in quantities 8g-38g. The compositions of CRG fibres used (MATT01 to 05) are set out below
   in Table 2.

**Table 2**

| **BATCH RECORD SELECTION** | | | | | | | | | | | | | | | | |
|---|---|---|---|---|---|---|---|---|---|---|---|---|---|---|---|---|
| Code | **Formulation as mole%** | | | | | | | | | | | | | **Solution Rates** | | **Physical Form** |
| | **Na**_{**2**}**O** | **CaO** | **Ag**_{**2**}**O** | **P**_{**2**}**O**_{**5**} | **MgO** | **K**_{**2**}**O** | **B**_{**2**}**O**_{**3**} | **MnO** | **Fe**_{**2**}**O**_{**3**} | **NaF** | **Ce**_{**2**}**O**_{**3**} | **Se** | **TOTAL** | **Annealed @ 37.5°C (mg.cm**^{**-2**}**.hr**^{**-1**}**)** | **Non-Annealed @ 37.5°C (mg.cm**^{**-2**}**.hr**^{**-1**}**)** | |
| MATT01 | | 27.98 | | 46.56 | 19.07 | | 6.36 | | | | | | 99.97 | N/A | N/A | R,F + C |
| MATT02 | | 30 | | 50 | 20 | | | | | | | | 100 | N/A | N/A | F,R + C |
| MATT03 | | 25 | | 50 | 20 | 5 | | | | | | | 100 | 0.0095 | 0.0151 | R + F |
| MATT04 | 26.05 | 17.6 | | 47.04 | | | 5.88 | 1.54 | 0.96 | 0.4 | 1 | | 100.47 | 0.0143 | 0.0165 | R + F |
| MATT05 | 25.19 | 17.03 | | 45.05 | | | 5.68 | 1.49 | 0.93 | 0.4 | 1.96 | 2.27 | 100 | 0.0177 | 0.02 | R + F |

| | | | | | | | | | | | | | | | | |
|---|---|---|---|---|---|---|---|---|---|---|---|---|---|---|---|---|
| R=rods; F=fibres; C=cullet | | | | | | | | | | | | | | | | |

### 4. Procedure

4.1 Test sample preparation
   4.1.1 Test samples were cut to the appropriate size (see section 4.2.1).
   4.1.2 Tissue culture polystyrene was employed as a negative control. The controls were not in the same physical form as the test material.
4.2 Fibres were examined in contact with the L929 cell line before any cleaning procedure. Fibres were examined in contact with both cell lines after cleaning in acetone, washing in PBS and sterilising in a dry oven at 190°C for 2 hours.
4.3 Cell preparation
   4.3.1 A cell subculture was prepared 24 hours before being introduced to the fibres.
4.4 Test procedure
   4.4.1 A small "bed" of the fibres was placed in the bottom of each well.
   4.4.2 The cell/medium preparation was gently pipetted onto the fibre bed.
   4.4.3 The 24-well plates were incubated and examined at 24 hours and 48 hours.
4.5 Interpretation of results
   4.5.1 At the conclusion of the incubation period the plates are removed from the incubator and examined under phase contract microscopy using x10 and x20 objective lenses.
   4.5.2 Each test and control material was initially evaluated using the scoring system detailed below. This evaluation was based on the appearance of the cells which were attached to the TCPS surface. It was not possible to carry out such an evaluation on the cells adhering to the fibres.

**Table 3 : Reactivity Responses**

| **Grade** | **Reactivity** | **Conditions of all cultures** |
|---|---|---|
| 0 | None | Discrete intracytoplasmic granules; no cell lysis |
| 1 | Slight | Not more than 20% of cells are round, loosely attached and without intracytoplasmic granules; occasional lysed cells are present |
| 2 | Mild | Not more than 50% of the cells are round and devoid of intracytoplasmic granules; extensive cell lysis and empty areas between cells |
| 3 | Moderate | No more than 70% of the cell layers contain rounded cells and/or are lysed |
| 4 | Severe | Nearly complete destruction of the cell layers |

### 4.6 Cytotoxicity Results

The following table highlights the results obtained following two separate tests. Two or four readings were taken at each test. In all cases negative control (TCPS) provided a 0 grade.

**Table 4**

| **Material Code** | **Grade Test 1 L9292** | **Material Code** | **Grade Test 2 L929** | **Test 2 HUE** |
|---|---|---|---|---|
| MATT01 | 0 | MATT01 | 0 | 0 |
| MATT02 | - | MATT02 | 0 | 0 |
| MATT03 | - | MATT03 | - | 0 |
| MATT04 | 0 | MATT04 | 0 | 0 |
| MATT05 | 0 | MATT05 | 0 | 0 |

### Comments

The results as detailed provide a very subjective assessment of material cytotoxicity. Where a grade 0 is shown, there was no evidence of toxicity and a confluent healthy monolayer of cells was present. Where there was evidence of contamination or where the cell monolayer is difficult to evaluate no score has been given.

### 4.7 Cell Substrate Results

The following table (Table 5) details the cell-fibre interactions and general cell culture conditions observed by phase contrast microscopy. As stated before phase-contrast images of the cells on the fibres are poor. A staining procedure was carried out with the HUE cells. This procedure uses a fluorescent staining technique (ethidium bromide and acridene orange) to identify cell viability. All observations were after 48 hour contact between cells and fibres.

**Table 5**

| | **MATT01** | **MATT02** | **MATT03** | **MATT04** | **MATT05** |
|---|---|---|---|---|---|
| L929 non- sterile fibres | Cells are viable. | Contamination | Contamination | Cells are viable. Healthy monolayer. There is some evidence of cell adhesion onto the fibres. | Cells are viable. Healthy monolayer. There is some evidence of cell adhesion onto the fibres. |
| L929 sterile fibres | Cells are viable but very granular. These fibres are having some adverse effect on the cells. | Culture medium pH levels are low. Cells are viable. There is no obvious cell adherence to the fibre. | pH is low. There seems to be evidence of contamination-though this may be degrading glass. Difficult to make any comment on cell viability. | Cells viable. Healthy monolayer. There is some evidence of cell adhesion onto the fibres. | Cells viable. Healthy monolayer. There is some evidence of cell the adhesion onto the fibres. |
| HUE sterile fibres | Cells are viable though granular in appearance. The medium pH has dropped. Some cells can be seen adhering to the fibres (Fig. 2). | Cells are viable. There is no obvious cell adherence to the fibre. | pH is low. There seems to be evidence of contamination- though this may be degrading glass. Difficult to make any comment on cell viability. | Cells viable. Cell monolayer on TCPS is healthy and equivalent to the control wells. There is some evidence of cell attachment but this is difficult to observe by phase contrast. (See Fig. 3). | Cells viable. Cell monolayer on TCPS is healthy and equivalent to the control wells. There is some evidence of cell attachment but this is difficult to observe by phase contrast. |

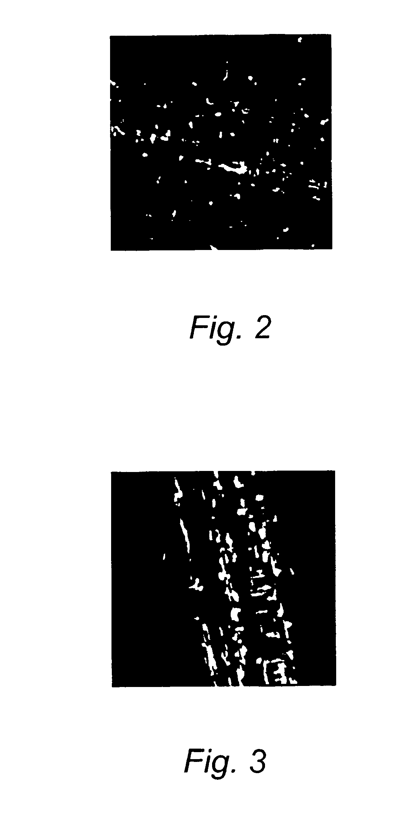

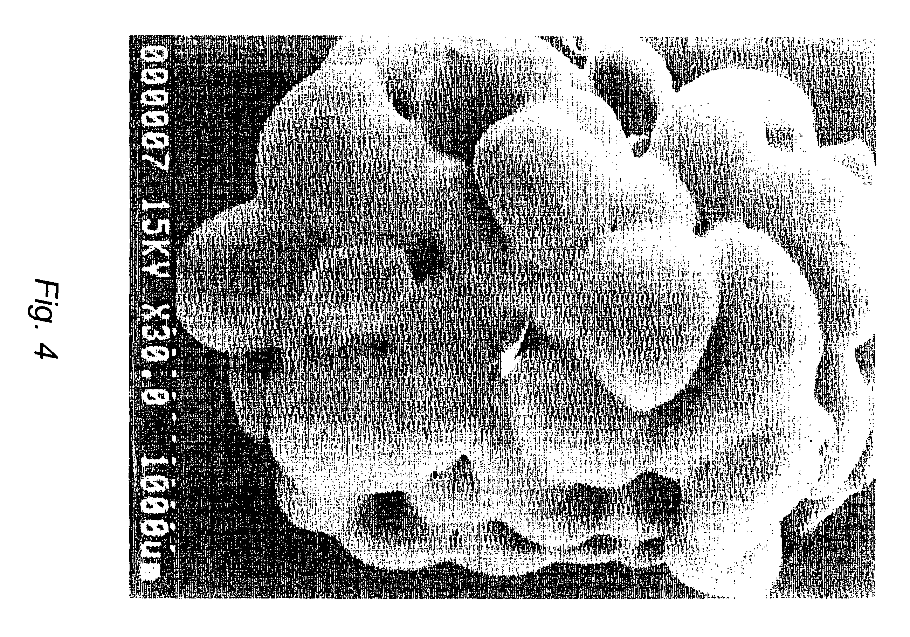

The images shown in Figs. 3 and 4 were obtained following the vital staining procedure and examined by fluorescent microscopy.

In Fig. 2 the bright areas represent viable cells (hue). The image shows an area with bundles of fibres radiating in many directions. In most cases the cells are rounded and not elongated on the fibres.

In Fig. 3 the bright areas represent viable cells (hue). Cells can be seen elongated on the fibres. In this image most of the fibres are oriented in the same direction. There is excellent cell coverage. This image is also representative of the result obtained with MATT05.

Of the five fibre compositions examined, MATT04 and MATT05 are providing an excellent substrate for cell adhesion. MATT01 has large numbers of cells adhering although the cell morphology is more rounded than that seen on the control surface. MATT02 and MATT03 show cells adhering but in much reduced numbers. There is no evidence of cytotoxicity with any of the fibres examined.

As well as demonstrating cell viability the procedure permitted a better evaluation of the cells attaching to the fibres. The cell-fibre interaction was much better than that indicated by phase contrast microscopy. It was noted that MATT04 and MATT05 had excellent cell adherence. MATT01 permitted a good cell adherence. There was cell attachment with MATT02 and MATT03 although this was poor in comparison with 01, 04 and 05.

### Example 3

A cell suspension (in complete cell culture medium supplemented with 5% foetal calf serum) at a concentration of approx. 5 x 10⁵ cell/ml was introduced to an established mouse fibroblast cell line (L929).

The material/cell interaction was examined using phase contrast microscopy at 24, 48 and 72 hours. In particular the following materials were examined (see Table 6 for composition of the glasses referred to by batch number).

### a) Glass sheet (flat); code 1051098-1

Cells can be seen adhering to the material and remain in contact with the material following sequential transfer between dishes. The cell morphology is rounded and the growth rate is considerably slower than observed with cells on the control dishes.
Nevertheless there is evidence of cell division taking place on the surface.

### b) Sintered glass beads (smooth surface); code BX-D221098-1, Sintered glass beads (rough surface); code BX-D221098-1

It is more difficult to make the observations with these samples using phase contrast. However, cells are
1 clearly present on the surface of both rough and smooth
2 samples. The cell population is certainly increasing
3 with time up to the 72 hour period. Again, this is
4 following sequential transfer at 24 hours.

**Table 6**

| **Batch Number** | **Formulation as mole%** | | | **Total** | **Solution Rates** | | **Physical Form** |
|---|---|---|---|---|---|---|---|
| | **Na**_{**2**}**O** | **CaO** | **P**_{**2**}**O**_{**5**} | | **Annealed @37.5°c (mg.cm'2 .hr'1** | **Annealed @37.5°c (mg.cm'2. hr'1** | |
| I051098 -1 | 25 | 28 | 47 | 100 | 0.0991 | 0.1364 | R+S |
| D221098 -1 | 11 | 42 | 47 | 100 | 0.0377 | 0.0446 | G+R |

| | | | | | | | |
|---|---|---|---|---|---|---|---|
| G=GRANULES R=RODS S=SHEETS | | | | | | | |

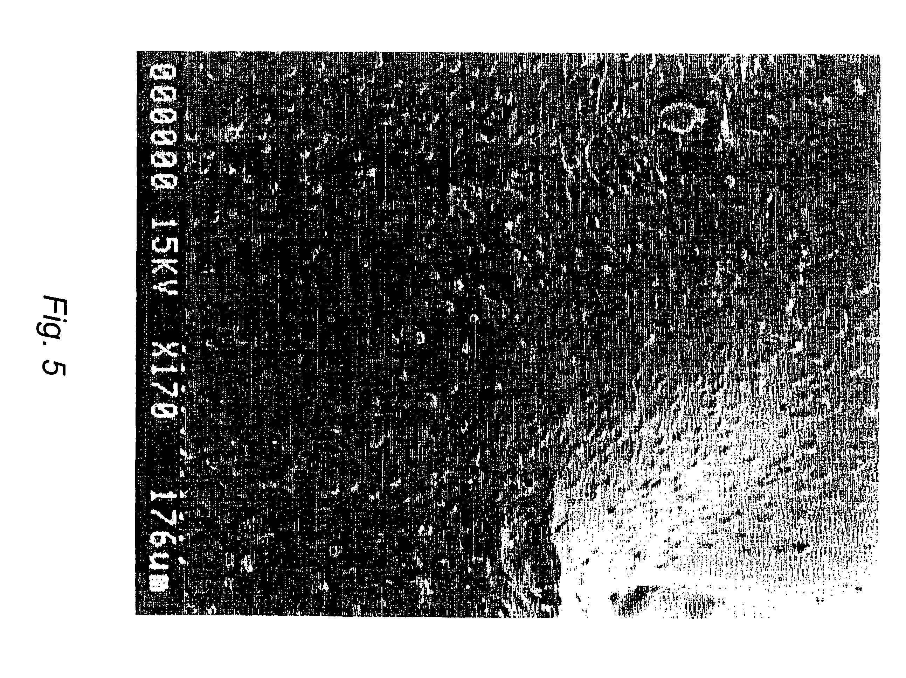

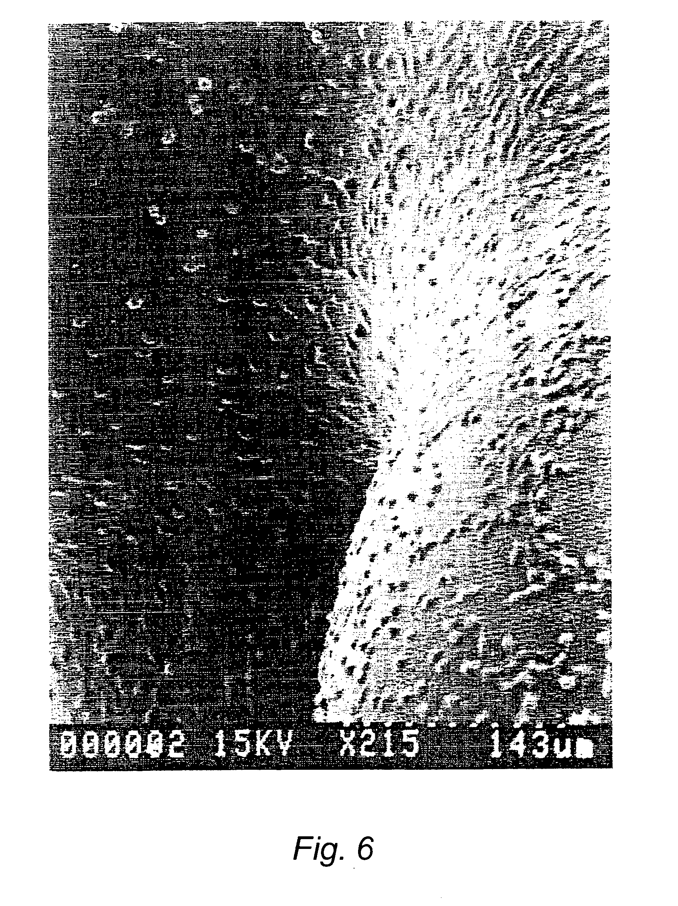

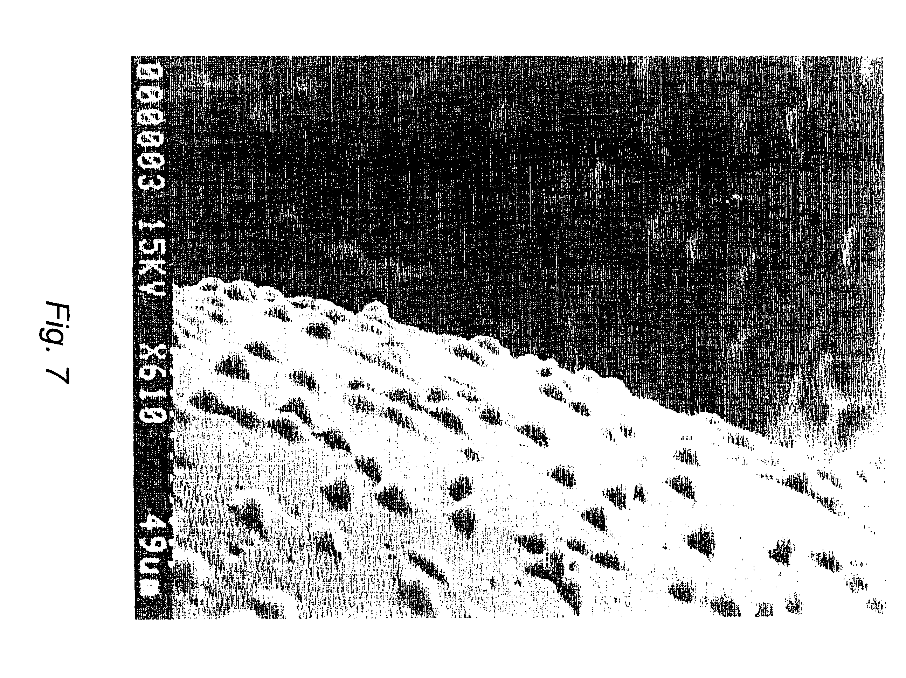

Sample SEMs were obtained (see Figs. 4 to 7) after cells had been in contact with the glass for 72 hours, fixed in 2.5% glutaraldehyde and dehydrated with alcohol. The samples were gold coated before viewing. The magnification is indicated on Figs. 4 to 7.

### Example 4

The potential for using CRG glass releasing two different kinds of metallic ions or boron as a cell culture growth substrate has been assessed for L929 mouse fibroblast cell line. To do so extraction vehicles were prepared which combined extracts of a mixture of two CRG's releasing a different type of metallic ions or boron, then it was determined at which concentration a positive effect on the metabolic activity of an L929 mouse fibroblast cell line was obtained. This data would give a good indication of the potential of these combined CRG as matrix for cell growth substrates according to the invention.

### Materials

The materials used in this investigation were control release glasses (CRG's) containing silver (Ag), copper (Cu), magnesium (Mg), zinc (Zn), nickel (Ni) ions and boron (B). The glasses were pre-ground to a particle size < 53 µm. The composition of the CRG's are shown in Table 7 below, along with information on dissolution rates. The extraction vehicles used were PBS (phosphate buffered solution - similar to fluids found in the body) and MEM (199 Modified Earles Medium - containing serum proteins).

**Table 7**

| CRG Composition and Dissolution Rates | | | | | | |
|---|---|---|---|---|---|---|
| **CRG** | **Na**_{**2**}**O (mol%)** | **CaO (mol%)** | **P**_{**2**}**O**_{**5**} **(mol%)** | **Ion (mol%)** | **Dissolution Rate (Mg/cm**^{**2**}**/hr) (annealed)** | **Dissolution Rate (Mg/cm**^{**2**}**/hr) (non-annealed)** |
| Ag | 32 | 18 | 47 | 3 | 0.5673 | 0.9413 |
| Cu | 31 | 15 | 47 | 7 | 0.6608 | 0.9973 |
| Mg | 38 | 9 | 47 | 6 | 0.9764 | 1.4004 |
| Zn | 31 | 14 | 47 | 8 | 1.5638 | 0.9232 |
| Ni | 32 | 18 | 47 | 3 | 0.2166 | 0.2999 |
| B | 41.5 | - | 41.5 | 17 | 0.1188 | 0.1744 |

### Establishing an L929 mouse fibroblast cell line

The cell line was established by subculturing an already existing cell line that was maintained by the University of Liverpool. The cells are maintained in 199 Modified Earles Medium (MEM), and stored in incubation at 37°C in a 5% CO₂/95% air atmosphere. The cells were grown to confluence in a flat-bottomed flask, and the monolayer was then harvested using trypsinization. The subculturing was carried out under sterile conditions using a laminar flow hood, and the following protocol was followed:

### Fibroblast Subculturing Protocol

- Take the original flask containing 10 ml of MEM, and check the cells under the microscope.
- Place the flask under the laminar flow hood and remove the MEM using a sterile glass pasteur and vacuum.
- Add 2.5 ml of 1% trypsin to the flask to loosen the confluent layer of cells. Observe the loosening of the cells under the microscope (it takes approx 3-4 mins).
- Once the cells are loosening (they take on a rounded appearance) return the flask to the hood and remove the trypsin using a pasteur and the vacuum, replace with 10 ml of fresh MEM.
- The flask is then agitated gently to remove the cells from the base, forming a suspension of approximately 10⁶ cells/ml concentration.
- 1 ml of this suspension is then added to 9 ml of fresh MEM in a fresh flask. Two flasks are prepared in this manner.
- Finally the flasks are labelled with name, date and cell line. The cells are then returned to the incubator and left for a week to establish a confluent layer of cells.

Subculturing then takes place once a week, with two subculturing flasks being prepared at a 1 in 10 concentration (then left for a week to develop a confluent layer), and the 96 well microtitre plates being prepared at a 1 in 40 concentration. The estimated cell concentration when the layer is confluent is 1 x 10⁶ cells/ml.

### Preparing the Extraction Vehicles

0.1 g of each of the CRG's making up the combinations was weighed out using a microbalance. The CRG's were placed into a sterile universal container and to this 20 ml of the extraction vehicle was added i.e. PBS or MEM. The extraction vehicles were then incubated at 37°C in a 5% CO₂/95% air atmosphere for a period of four hours. Four hours was selected as the CRG's had completely dissolved in the PBS within this time, and began to precipitate out if left for longer. The CRG's had still not dissolved fully when left in MEM for a period of 72 hours, and so it was decided to use four hours for both extraction vehicles. After this time they were removed from the incubator and filtered under sterile conditions. The filtering was performed to remove any contamination and precipitates.

The next step was to prepare the range of concentrations. The filtered extract (20 ml) was initially added to 20 ml of double strength culture medium to provide a starting concentration of 50% in a single strength medium. This concentration was not used during the tests. Next, 10 ml of MEM was measured out into a fresh universal container and 10 ml of 50% solution added to provide a 25% concentration, this was further diluted in MEM until a concentration of 0.05% had been established.
1. 10 ml 50.0% solution + 10 ml fresh MEM = 25.0% concentration*.
   * Concentrations used throughout the test.
2. 10 ml 25.0% solution + 10 ml fresh MEM = 12.5% concentration*.* Concentrations used throughout the test.
3. 10 ml 12.5% solution + 10 ml fresh MEM = 6.25% concentration*.* Concentrations used throughout the test.
4. 10 ml 6.25% solution + 10 ml fresh MEM = 3.12% concentration.
5. 10 ml 3.12% solution + 10 ml fresh MEM = 1.60% concentration*.* Concentrations used throughout the test.
6. 10 ml 1.60% solution + 10 ml fresh MEM = 0.80% concentration.
7. 10 ml 0.80% solution + 10 ml fresh MEM = 0.40% concentration*.* Concentrations used throughout the test.
8. 10 ml 0.40% solution + 10 ml fresh MEM = 0.20% concentration.
9. 10 ml 0.20% solution + 10 ml fresh MEM = 0.10% concentration*.
   * Concentrations used throughout the test.
10. 10 ml 0.10% solution + 10 ml fresh MEM = 0.05% concentration*.
   * Concentrations used throughout the test.

### Setting up the well plates

The well plates were seeded with a 1 in 40 concentration of fibroblasts. The medium was pipetted into the wells using a pipetter and a sterile trough. A 1 in 40 concentration was chosen as the effects on the growth of the cells was being investigated and so a confluent layer was not required.

The plates were then re-incubated at 37°C in a 5%/CO₂/95% air atmosphere) for a period of 48 hours or 96 hours. After this time, the medium was removed from the plates under sterile conditions and replaced with the prepared exudates in the following manner. Twelve wells were used for each concentration, and again the pipetter and sterile troughs were used to deliver the extraction of vehicles to the wells.

| | | | |
|---|---|---|---|
| Control | 12.5% | 1.6% | 0.1% |
| 12 wells | 12 wells | 12 wells | 12 wells |
| 25% | 6.25% | 0.4% | 0.05% |
| 12 wells | 12 wells | 12 wells | 12 wells |

The plates were then incubated (at 37°C in a 5% CO₂/95% air atmosphere) for a further period of 48 hours or 72 hours, after which the extraction vehicle was removed and an MTT assay was performed.

### Performing the MTT Assay

The MTT assay is a rapid colomeric assay based on the tetrazolium salt MTT (3- (4,5-dimethylthiazol-2-yl)-2,5-diphenyl tetrazolium bromide). The cells produce certain enzymes when they are growing, and the MTT salt solution is cleaved into blue formazan crystals by one such enzyme reportedly the "mitochondrial enzyme succinate-dehydrogenase". The crystals are soluble in iso-propranol and produce a coloured aqueous solution. The amount of formazan produced is said to be proportional to the number of viable cells present i.e. the darker the shade of blue produced indicates a greater level of cell activity.

200 µl of MTT salt solution was added to each well in the plates at a concentration of 1 mg/ml and the plates were then incubated at 37°C for a period of four hours. The MTT solution was then removed and replaced with approximately 100 µl of iso-propranol. The plates were then incubated for a further 20 minutes (at 37°C). To ensure complete dissolution of the blue formazan crystals the plates were gently shaken.

The final stage was to measure the optical density readings of the plates using an enzyme-linked immunosorbent assay (ELISA) plate reader at a test wavelength of 570 nm.

The repetition for the results was obtained by using 12 wells in the 96 well plate for each of the concentrations and the control. Repetition was required to reduce the amount of error involved with the results.

So that the results were comparable, various different steps were taken. These included:
- The dissolution time for each extraction vehicle for four hours.
- The incubation times were the same for each exudate, either two days in MEM followed by two days with the exudate (2d-2d), or four days in MEM followed by three days with the exudate (4d-3d).
- The cells were kept at constant conditions throughout the investigation, 37°C/5% CO₂.

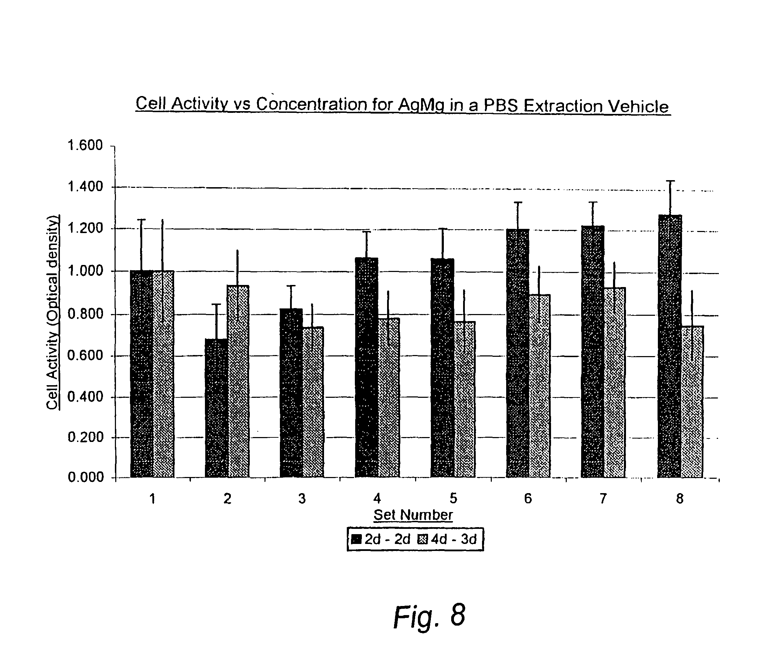

The results obtained have been displayed as bar charts and are shown in Figs. 8 to 20. The important features to consider on the charts are the set numbers and the cell activity levels. Each of the set number correlates to a different concentration as follows:

| Set One | : Control |
|---|---|
| Set Two | : 25% |
| Set Three | : 12.5% |
| Set Four | : 6.25% |
| Set Five | : 1.6% |
| Set Six | : 0.4% |
| Set Seven | : 0.1% |
| Set Eight | : 0.05% |

The control used throughout the study was cells at a 1 in 40 concentration proliferating in 5% MEM with added fetal calf serum and antibiotics. This control was used because it gave a good indication of the accuracy of the MTT assay, and could be easily used to determine whether the combinations were antagonising or synergising cell growth.

To obtain the cell activity level from the optical density readings the control was fixed to the level of one, the remainder of the optical density readings obtained were then adjusted in accordance with this level. This means that by observing the charts it is easy to determine whether the combinations are having a positive or negative effect on the cell growth.

Several of the optical density readings could not be counted by the ELISA plate when the cells were left for the longer period of time. The amount of cell activity was greater than the range of the plate reader. However, these results are known to be at least three, and so have been added to the results as this minimum value. Results where this has occurred are marked with an * on the charts.

### Results

### Ag/Mg in a PBS Extraction Vehicle (Fig. 8)

The graph of Fig. 8 shows a stimulation response evident for the 2d-d2 sample over the range 6.25% to 0.05%, with the peak stimulation occurring at a concentration of 0.05%, exhibiting a 28% increase on the control level.

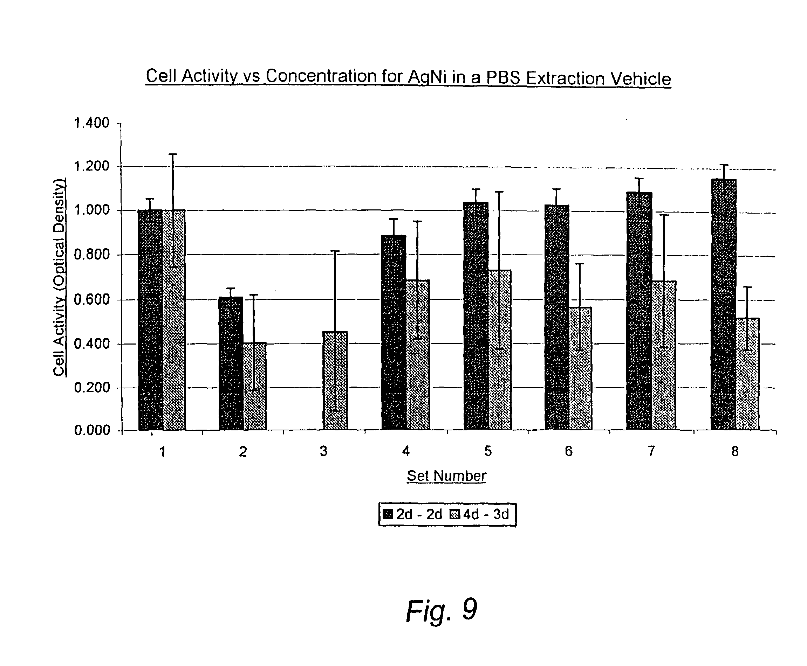

### Ag/Ni in a PBS Extraction Vehicle (Fig. 9)

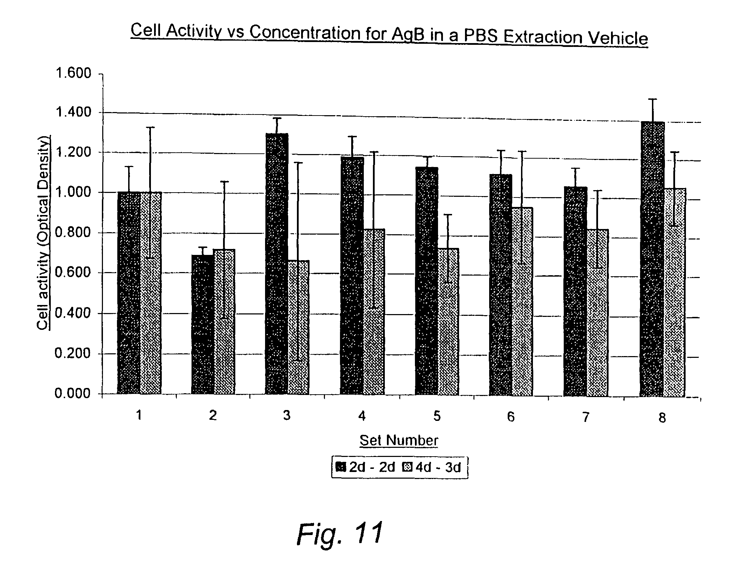

The graph shown in Fig. 11 shows a stimulatory effect occurring during the 2d-2d period. There is a stimulation taking place at 0.05%, and being 15% above the control. No result was obtained for 12.5% for 2d-2d as it was not initially selected in the range of concentrations.

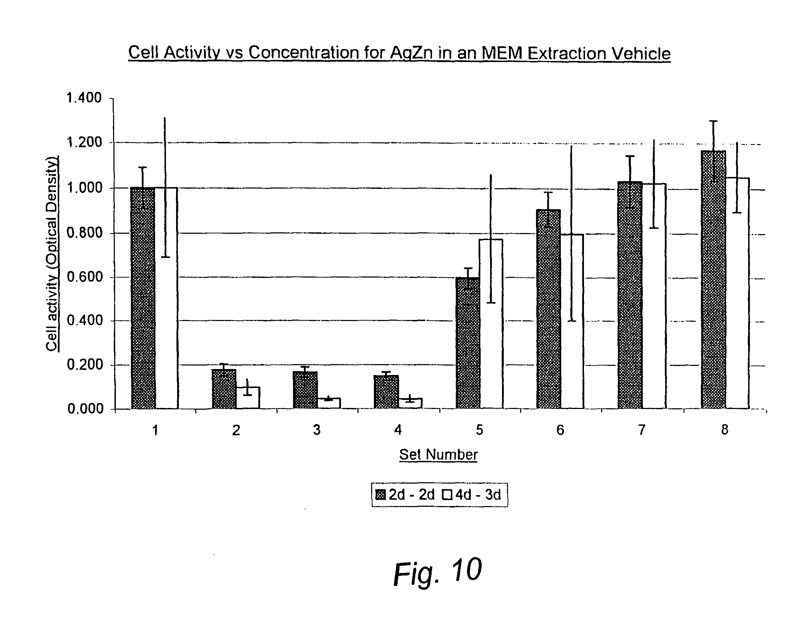

### Ag/Zn in an MEM Extraction Vehicle (Fig. 10)

There is evidence of this combination reacting toxically to the cells at high concentrations (25%, 12.5% and 6.25%) for both length of times. However, for the lowest concentrations of 0.1% and 0.05% cell proliferation has been induced. The stimulation for the shorter time period is greater than for 4d-3d, with a 17% increase on the control level at 0.05%.

### Ag/B in a PBS Extraction Vehicle (Fig. 11)

The analysis of the graph represented in Fig. 11 shows that for 2d-2d periods the stimulation of the cell metabolism begins at 12.5%, with the peak occurring at 0.05%, 40% above the control. For the 4d-3d period, a stimulatory effect of 5% above the control occurs at the 0.05% concentration.

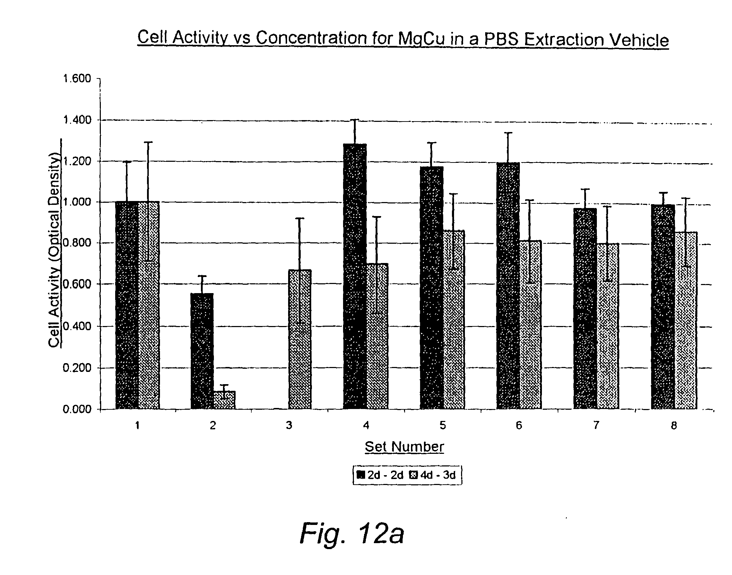

### Mg/Cu in a PBS Extraction Vehicle (Fig. 12a)

The graph of Fig. 12a shows that a stimulatory response occurs for the 2d-2d time period for concentrations between 6.25% and 0.4% with a peak being displayed at 6.25%, indicating a 28% increase on the control.

Considering the ions individually, magnesium is known to be fairly non-toxic ion even in the high concentrations, however, copper ions are extremely toxic at high concentrations. This would suggest that by combining the copper with the magnesium, the magnesium is suppressing the affect of the copper over this particular range for the PBS.

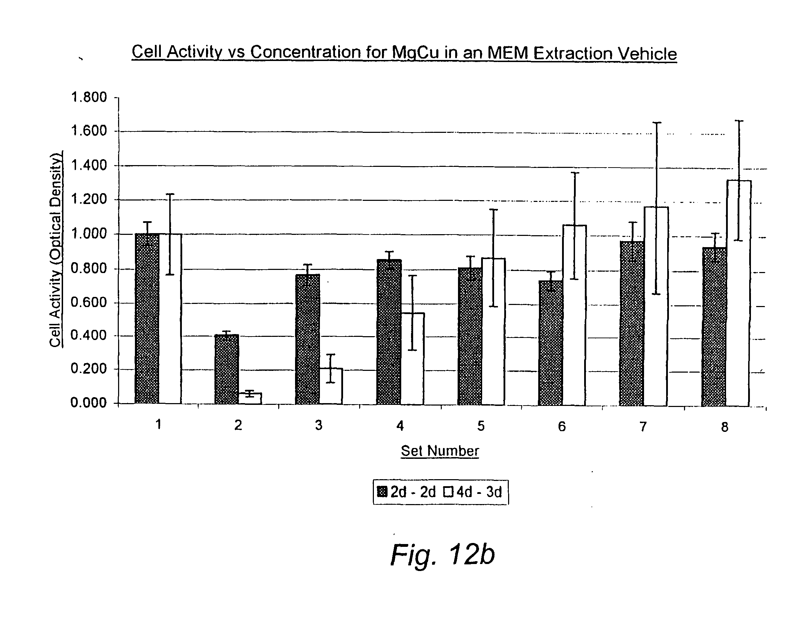

### Mg/Cu in an MEM Extraction Vehicle (Fig. 12b)

The graph of Fig. 12b shows that cell proliferation occurs at the lower concentrations, (0.4%, 0.1% and 0.05%) for the 4d-3d samples. A peak occurs at 0.05% indicating a 33% increase on the control levels of cells. The 4d-3d time period also exhibits a gradual increase from toxicity to stimulation.

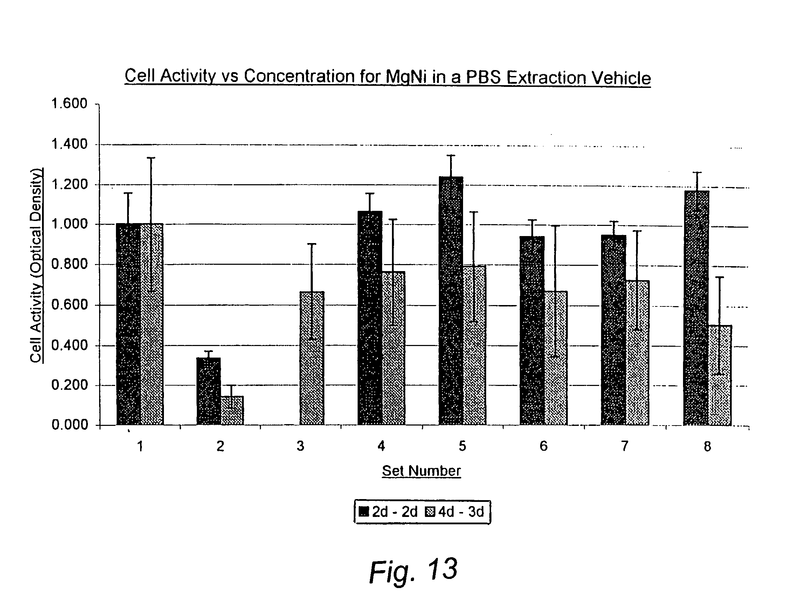

### Mg/Ni in a PBS Extraction Vehicle (Fig. 13)

The 2d-2d time period exhibits very interesting behaviour with a stimulatory peak occurring at a concentration of 1.6%, indicating a 24% increase on cell metabolism above the control. A stimulatory response also occurs at 6.25% and 0.05%.

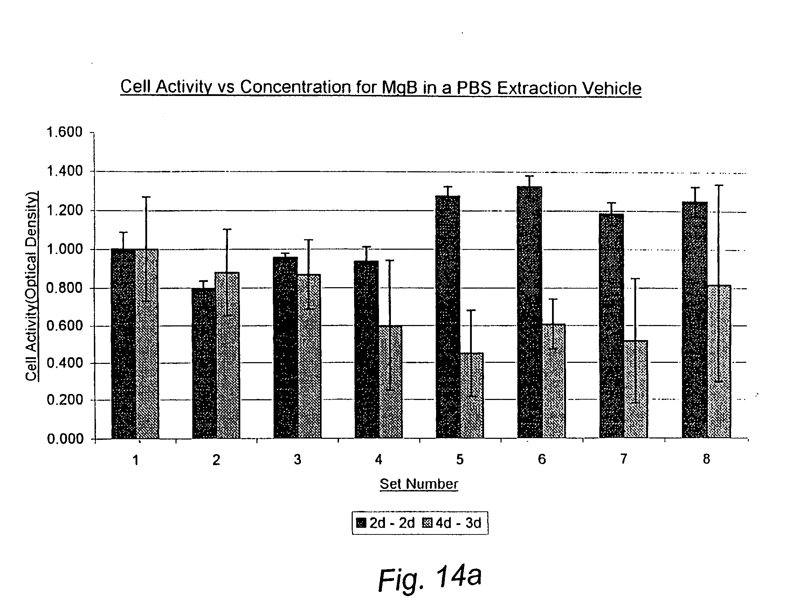

### Mg/B in a PBS Extraction Vehicles (Fig. 14a)

This CRG combination produces a significant stimulatory effect for the 2d-2d period, with stimulation being apparent from 0.05% up to 1.6% (although 0.1% is just below the control). The peak in this positive effect occurs at a concentration of 1.6% and is 32% above the control level.

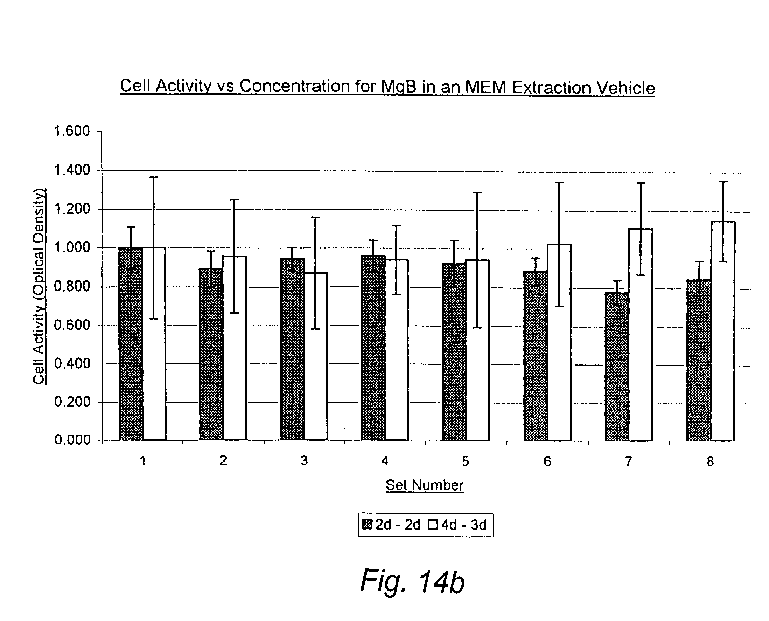

### Mg/B in an MEM Extraction Vehicle (Fig. 14b)

The graph of Fig. 14b shows that there is a very small toxic effect on the metabolism of the cells in both of the chosen time periods. In fact the cell activity levels for concentrations between 25% and 1.6% are fairly equal. For the 4d-3d time, a stimulatory effect is present from 0.4% onwards. The maximum stimulation occurs at 0.05% and is 14% above the control.

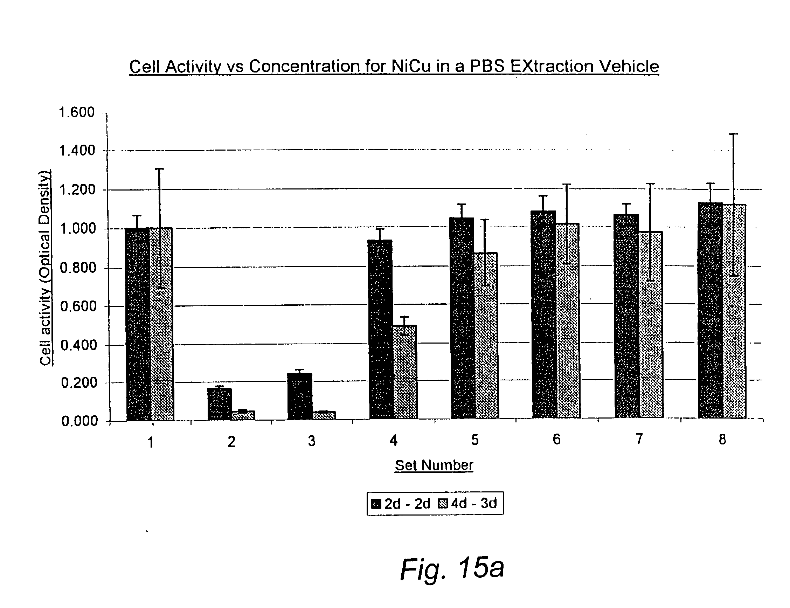

### Ni/Cu in a PBS Extraction Vehicle (Fig. 15a)

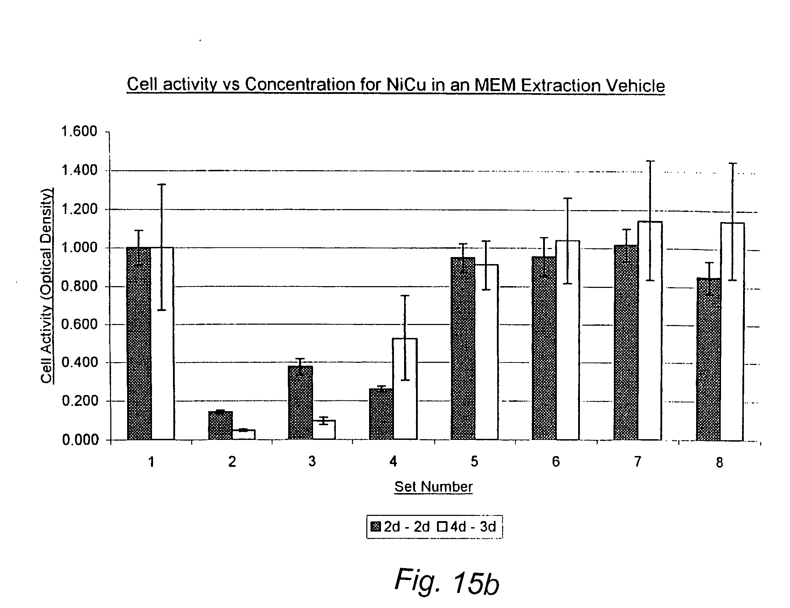

The graph of Fig. 15a shows high levels of toxicity occurring at the high concentrations 25% and 12.5% for both lengths of time. For the 2d-2d time there is a sudden increase in the cell activity to just 7% below the control at the 6.25% CRG concentration. Cell metabolism stimulation can be seen from 1.6% onwards for 2d-2d, and 0.4% for the 4d-3d. Both time periods peak at 0.05%, at approximately the same level 12/13%. Ni/Cu in an MEM Extraction Vehicle (Fig. 15b)

A toxic effect can be seen at the high concentrations for both time periods as with many of the other combinations. The toxicity levels are then significantly lower at 1.6%. For the 4d-3d time there is evidence of stimulation from 0.4% through to 0.05%, with both 0.1% and 0.05% stimulating by 15% above the control. There is a sign of stimulation in the 2d-2d time at 0.1%, however, it is only 1.5% above the level of the control.

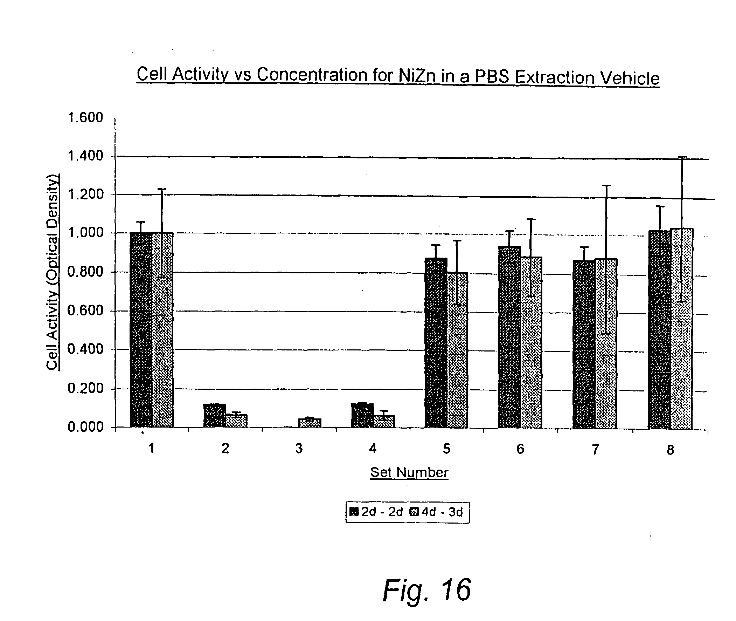

### Ni/Zn in an MEM Extraction Vehicle (Fig. 16)

The main feature of the graph of Fig. 16 is the stimulation peak for the 2d-2d time at a concentration of 0.1%, 12% above the control. The stimulation peak for 4d-3d at the 0.05% concentration approximately 8% above the control, and toxic effects are present at the high concentrations. There is a sharp increase in the cell activity between 6.25% and 1.6%.

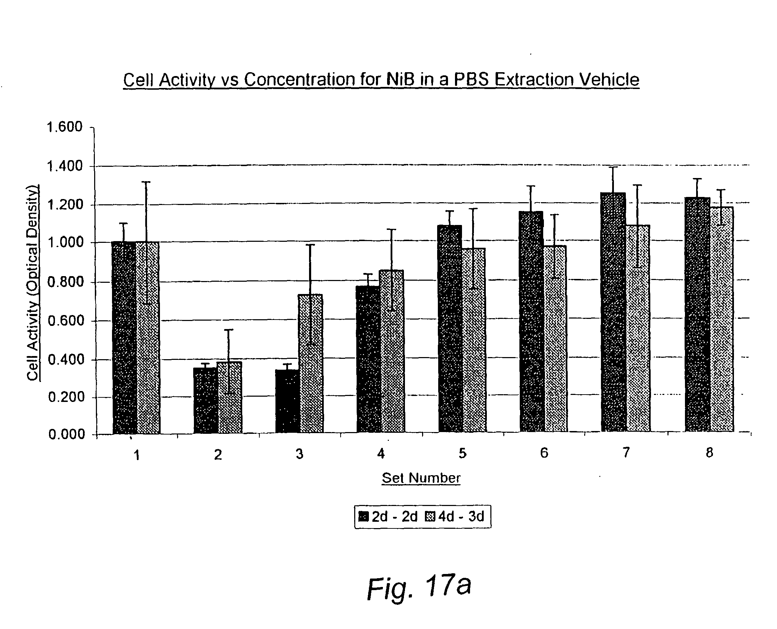

### Ni/B in a PBS Extraction Vehicle (Fig. 17a)

This combination elicits a stimulatory response on the cell metabolism over the concentration range 1.6% to 0.05% for the 2d-2d with a maximum occurring at a concentration of 0.1%, 25% above the control. There is a gradual increase in the activity of the cells over the 4d-3d period, with non-toxicity being reached at 0.1%, and a peak occurring at 0.05%, 17% above the control.

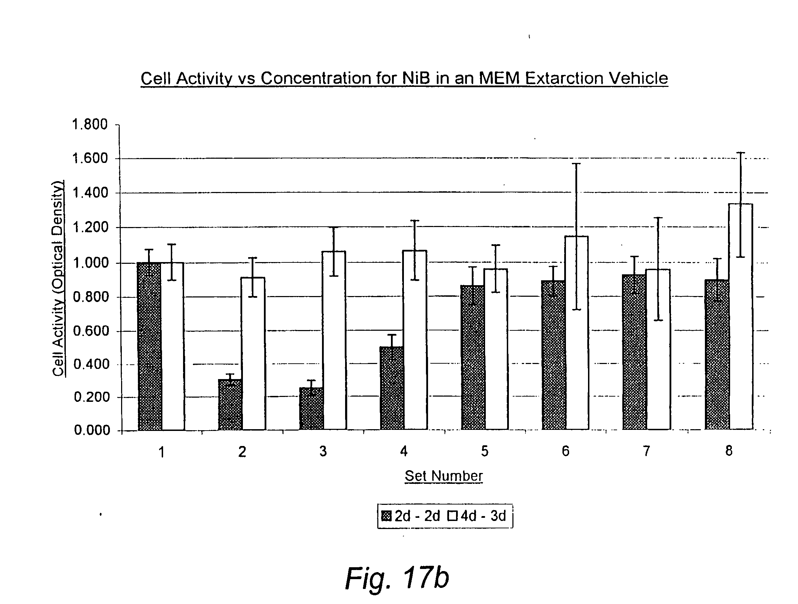

### Ni/B in an MEM Extraction Vehicle (Fig. 17b)

The results shown in the graph of Fig. 17b for the 2d-2d period are high toxicity at the high concentrations with a gradual increase in the cell activity over the range. There is a positive effect occurring due to this combination. There is stimulation from 12.5% onwards, peaking at 0.05%, 33% above the control.

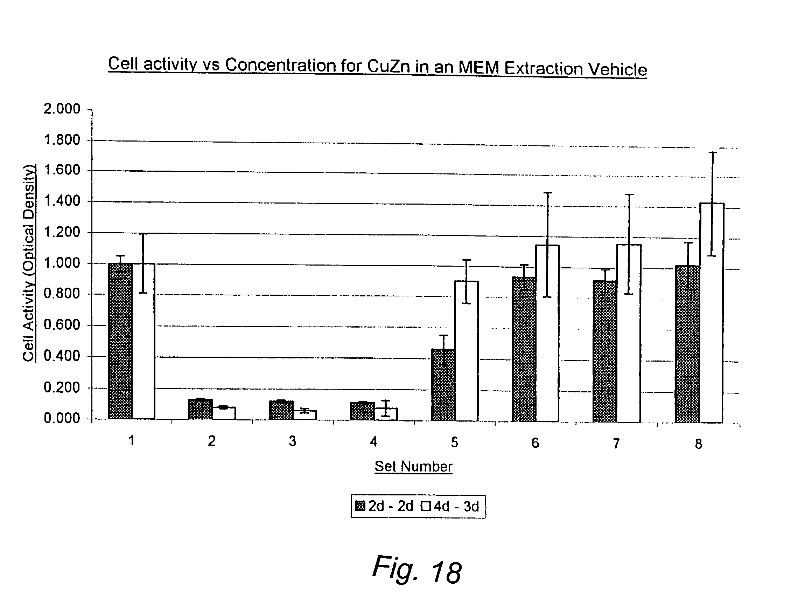

### Cu/Zn in an MEM Extraction Vehicle (Fig. 18)

The graph in Fig. 18 shows that this combination elicits a toxic response at the higher concentrations (25% through to 6.25%). The cell growth ceases to be affected at 0.05% for the 2d-2d time. There is cell proliferation occurring in the 4d-3d set of results from 0.4% onwards. The maximum stimulation occurs at 0.05%, with a significant increase of 42% on the control level.

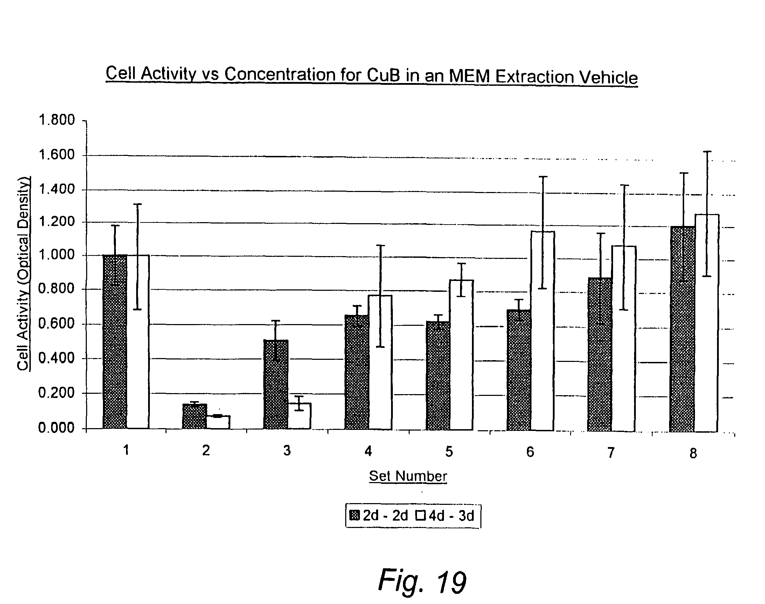

### Cu/B in an MEM Extraction Vehicle (Fig. 19)

Considering the graph of Fig. 19, it is noticeable that there is a gradual increase in the cell activity for both 2d-2d and 4d-3d. At 25% the combination elicits a toxic effect on the cells and at 0.05% it produces cell proliferation. It is 20% above the control for 2d-2d, and 28% above the control for the 4d-3d.

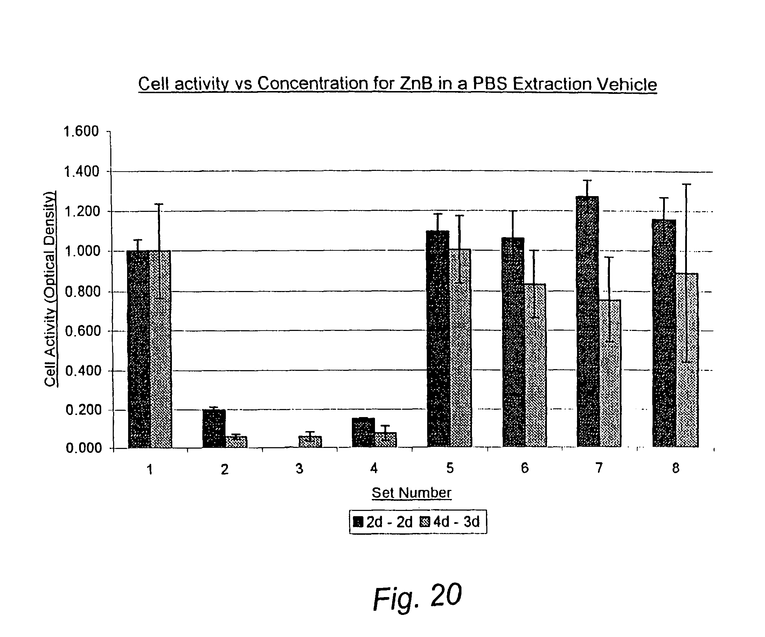

### Zn/B in a PBS Extraction Vehicle (Fig. 20)

Considering the graph of Fig. 20 there is evidence of toxicity at the high concentrations (25%, 12.5% and 6.25%) for both time periods. The stimulatory effect by the cells to this combination for the 2d-2d period begins at a concentration of 1.6% and peaks at 0.1%, 27% greater than the control level.

### Conclusion

The results collected show that CRG releasing various combinations of metallic ions and boron have potential as a matrix in a cell culture growth substrate according to the invention. This is the case in particular for combinations containing boron where in some combinations the stimulation is 25% greater than the control.

## Claims

1. A cell culture growth substrate adapted to sustain growth of living cells, said substrate comprising a water soluble glass matrix which comprises at least a portion of its surface coated with living cells, wherein the glass of said water soluble glass matrix comprises at least one metallic ion or boron containing compound.

2. The substrate of Claim 1, wherein a portion of the surface of said substrate is coated with living cells.

3. The substrate of either one of Claims 1 and 2, wherein the water soluble glass is a phosphate glass.

4. The substrate of any one of Claims 1 to 3, wherein said water soluble glass comprises phosphorous pentoxide as glass former.

5. The substrate of any one of Claims 1 to 4, wherein said glass comprises an oxide or a carbonate of an alkali metal, an alkaline earth metal or a transition metal as glass modifier.

6. The substrate of Claim 5, wherein said glass modifier is sodium oxide, potassium oxide, magnesium oxide, zinc oxide or calcium oxide.

7. The substrate of any one of Claims 1 to 6, wherein said glass has a dissolution rate ranging from substantially zero to 2.0 mg/cm²/hour at 38°C.

8. The substrate of any one of Claims 1 to 7, wherein said glass enables a controlled release of additives in an aqueous medium.

9. The substrate of Claim 8, wherein said additives comprise at least one metallic ion or boron containing compound.

10. The substrate of any one of Claims 1 to 9, wherein said water soluble glass matrix comprises water soluble glass fibres.

11. The substrate of Claim 10, wherein said glass fibres are sintered together to form a non-woven mat.

12. The substrate of any one of Claims 1 to 9, wherein said water soluble glass matrix comprises finely comminuted glass particles.

13. The substrate of Claim 12, wherein said finely comminuted glass particles are sintered together to form a porous structure.

14. The substrate of either one of Claims 12 and 13, wherein said glass particles have an average diameter of from 15 microns to 6 mm.

15. The substrate of any one of Claims 1 to 14 for use as an implant to replace or promote repair of damaged tissue in a patient.

16. A method to encourage growth of living tissue *in vitro,* said method comprising the step of providing the substrate of Claims 1 to 14.

17. The method of Claim 16, wherein said method includes the step of delivering metal ions or boron to an aqueous medium at a rate which maintains a concentration of metal ions or boron in said aqueous medium of not less than 0.01 parts per million and not greater than 10 parts per million.

## Patentansprüche

1. Ein Zellkulturwachstumssubstrat, das ausgeführt ist, um das Wachstum lebender Zellen zu erhalten, wobei das Substrat eine wasserlösliche Glasmatrix beinhaltet, die zumindest einen Abschnitt ihrer Oberfläche beinhaltet, der mit lebenden Zellen bedeckt ist, wobei das Glas aus der wasserlöslichen Glasmatrix zumindest eine ein Metallion oder Bor enthaltende Verbindung beinhaltet.

2. Substrat gemäß Anspruch 1, wobei ein Abschnitt der Oberfläche des Substrats mit lebenden Zellen bedeckt ist.

3. Substrat gemäß Anspruch 1 oder 2, wobei das wasserlösliche Glas ein Phosphatglas ist.

4. Substrat gemäß einem der Ansprüche 1 bis 3, wobei das wasserlösliche Glas Phosphorpentoxid als Glasbildner beinhaltet.

5. Substrat gemäß einem der Ansprüche 1 bis 4, wobei das Glas ein Oxid oder ein Karbonat eines Alkalimetalls, eines Erdalkalimetalls oder eines Übergangsmetalls als Glaswandler beinhaltet.

6. Substrat gemäß Anspruch 5, wobei der Glaswandler Natriumoxid, Kaliumoxid, Magnesiumoxid, Zinkoxid oder Kalziumoxid ist.

7. Substrat gemäß einem der Ansprüche 1 bis 6, wobei das Glas eine Auflösungsgeschwindigkeit in dem Bereich von im Wesentlichen Null bis 2,0 mg/cm²/Stunde bei 38 °C aufweist.

8. Substrat gemäß einem der Ansprüche 1 bis 7, wobei das Glas eine kontrollierte Freisetzung von Zusatzstoffen in einem wässrigen Medium ermöglicht.

9. Substrat gemäß Anspruch 8, wobei die Zusatzstoffe zumindest eine ein Metallion oder Bor enthaltende Verbindung beinhalten.

10. Substrat gemäß einem der Ansprüche 1 bis 9, wobei die wasserlösliche Glasmatrix wasserlösliche Glasfasern beinhaltet.

11. Substrat gemäß Anspruch 10, wobei die Glasfasern zusammengesintert sind, um eine Vliesmatte zu bilden.

12. Substrat gemäß einem der Ansprüche 1 bis 9, wobei die wasserlösliche Glasmatrix stark verkleinerte Glaspartikel beinhaltet.

13. Substrat gemäß Anspruch 12, wobei die stark verkleinerten Glaspartikel zusammengesintert sind, um eine poröse Struktur zu bilden.

14. Substrat gemäß Anspruch 12 oder 13, wobei die Glaspartikel einen durchschnittlichen Durchmesser von 15 Mikrometer bis 6 mm aufweisen.

15. Substrat gemäß einem der Ansprüche 1 bis 14 zur Verwendung als Implantat, um beschädigtes Gewebe eines Patienten zu ersetzen oder seine Wiederherstellung zu fördern.

16. Ein Verfahren zur Anregung des Wachstums lebenden Gewebes *in vitro,* wobei das Verfahren den Schritt des Bereitstellens des Substrats aus Ansprüchen 1 bis 14 beinhaltet.

17. Verfahren gemäß Anspruch 16, wobei das Verfahren den Schritt des Zuführens von Metallionen oder Bor an ein wässriges Medium mit einer Geschwindigkeit, die eine Metallionen- oder Borkonzentration in dem wässrigen Medium von nicht weniger als 0,01 Teilen je Million und nicht mehr als 10 Teilen je Million beibehält, umfasst.

## Revendications

1. Un substrat de croissance pour culture cellulaire adapté pour soutenir la croissance de cellules vivantes, ledit substrat comportant une matrice en verre soluble dans l'eau dont au moins une portion de sa surface est revêtue de cellules vivantes, dans lequel le verre de ladite matrice en verre soluble dans l'eau comporte au moins un composé contenant un ion métallique ou du bore.

2. Le substrat de la revendication 1, dans lequel une portion de la surface dudit substrat est revêtue de cellules vivantes.

3. Le substrat de l'une ou l'autre des revendications 1 et 2, dans lequel le verre soluble dans l'eau est un verre phosphate.

4. Le substrat de n'importe laquelle des revendications 1 à 3, dans lequel ledit verre soluble dans l'eau comporte du pentoxyde de phosphore en tant que vitrifiant.

5. Le substrat de n'importe laquelle des revendications 1 à 4, dans lequel ledit verre comporte un oxyde ou un carbonate d'un métal alcalin, d'un métal alcalinoterreux ou d'un métal de transition en tant que modificateur de verre.

6. Le substrat de la revendication 5, dans lequel ledit modificateur de verre est de l'oxyde de sodium, de l'oxyde de potassium, de l'oxyde de magnésium, de l'oxyde de zinc ou de l'oxyde de calcium.

7. Le substrat de n'importe laquelle des revendications 1 à 6, dans lequel ledit verre a un taux de dissolution compris dans la gamme allant de substantiellement zéro à 2,0 mg/cm²/heure à 38° C.

8. Le substrat de n'importe laquelle des revendications 1 à 7, dans lequel ledit verre permet une libération contrôlée d'additifs dans un milieu aqueux.

9. Le substrat de la revendication 8, dans lequel lesdits additifs comportent au moins un ion métallique ou un composé contenant du bore.

10. Le substrat de n'importe laquelle des revendications 1 à 9, dans lequel ladite matrice en verre soluble dans l'eau comporte des fibres de verre solubles dans l'eau.

11. Le substrat de la revendication 10, dans lequel lesdites fibres de verre sont frittées ensemble pour former un tapis non tissé.

12. Le substrat de n'importe laquelle des revendications 1 à 9, dans lequel ladite matrice en verre soluble dans l'eau comporte des particules de verre finement broyées.

13. Le substrat de la revendication 12, dans lequel lesdites particules de verre finement broyées sont frittées ensemble pour former une structure poreuse.

14. Le substrat de l'une ou l'autre des revendications 12 et 13, dans lequel lesdites particules de verre ont un diamètre moyen allant de 15 microns à 6 mm.

15. Le substrat de n'importe laquelle des revendications 1 à 14 destiné à être utilisé comme implant pour remplacer du tissu endommagé chez un patient ou en favoriser la réparation.

16. Une méthode pour encourager la croissance de tissu vivant in vitro, ladite méthode comportant l'étape de fournir le substrat des revendications 1 à 14.

17. La méthode de la revendication 16, dans laquelle ladite méthode comprend l'étape de fournir des ions métalliques ou du bore à un milieu aqueux à un taux qui maintient une concentration d'ions métalliques ou de bore dans ledit milieu aqueux ne tombant pas en dessous de 0,01 partie par million et ne dépassant pas 10 parties par million.
